# DEMANDE DE BREVET EUROPEEN

(11) **EP 0 655 452 A1**
(43) Date de publication de la demande: **31.05.1995**
(21) Numéro de dépôt: 94402641.8
(22) Date de dépôt: 21.11.1994
(51) Int. Cl.: C07F 5/02

(54) **Dérivés de triarylborane, leur préparation et leur utilisation comme intermédiaires de synthèse**

(30) Priorité: 26.11.1993 FR 9314152
(71) Demandeur: SYNTHELABO, F-92350 Le Plessis Robinson (FR)
(72) Inventeur: Chekroun, Isaac, F-93800 Epinay (FR); Rossey, Guy, F-78960 Voisins le Bretonneux (FR); Magnat, Michel, F-78300 Poissy (FR)
(74) Mandataire: Thouret-Lemaitre, Elisabeth

(57) **Abrégé**

Dérivés de triarylborane répondant à la formule (1) 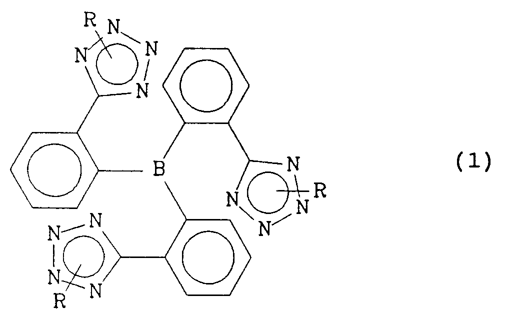
dans laquelle
R représente soit un groupe -CR₁R₂R₃ où R₁, R₂ et R₃ sont chacun indépendamment l'un de l'autre, un groupe (C₁-C₂)alkyle ou aryle, soit un groupe -CH₂OR₄ où R₄ est un groupe (C₁-C₂)alkyle ou benzyle, soit un groupe -Si(R₅)₃ où R₅ est un groupe (C₁-C₂) alkyle ou aryle, R étant en position 1 ou 2 du cycle tétrazole,
leur préparation et leur utilisation comme intermédiaires de synthèse.

## Description

La présente invention concerne des dérivés de triarylborane répondant à la formule (1) 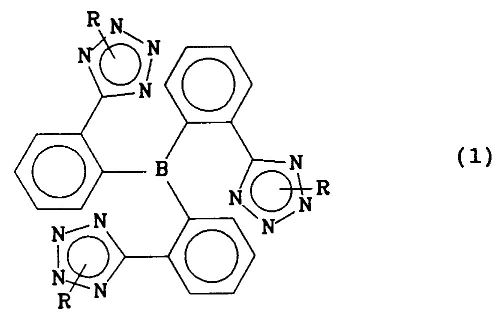
dans laquelle
R représente soit un groupe -CR₁R₂R₃ où R₁, R₂ et R₃ sont chacun indépendamment l'un de l'autre, un groupe (C₁-C₂)alkyle ou aryle, soit un groupe -CH₂OR₄ où R₄ est un groupe (C₁-C₂)alkyle ou benzyle, soit un groupe -Si(R₅)₃ où R₅ est un groupe (C₁-C₂) alkyle ou aryle, R étant en position 1 ou 2 du cycle tétrazole,
leur préparation et leur utilisation dans des couplages aryle-aryle, aryle-naphtyle, aryle-hétéroaryle ou aryle-hétéroaryle condensé en présence de catalyseurs à base de métaux de transition et, en particulier, de palladium.

Les composés préférés selon l'invention sont les composés de formule (1) dans laquelle R représente un groupe -CR₁R₂R₃ où R₁, R₂ et R₃ sont chacun indépendamment l'un de l'autre un groupe (C₁-C₂) alkyle.

Parmi ceux-ci le composé particulièrement préféré est le composé répondant à la formule (1) dans laquelle R est le groupe 1,1-diméthyléthyle.

Conformément à l'invention, les composés de formule (1) peuvent être préparés à partir de dérivés de formule (2) dans laquelle R est tel que défini précédemment et Z représente soit un atome d'hydrogène, soit un atome d'halogène tel que par exemple un atome de brome, de chlore ou d'iode, selon le schéma ci-après.

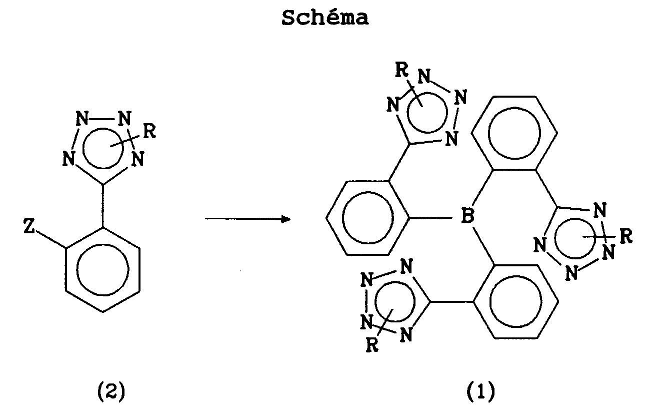

On fait réagir le composé de formule (2) avec un alkyllithium, tel le n-butyllithium ou l'hexylli-thium ou l'hexaméthyldisilazane lithié, dans un solvant aprotique comme le tétrahydrofurane, à une température comprise entre 20 °C et la température de reflux. On obtient un organolithien que l'on fait réagir avec un tri(C₁-C₄)alkylborate ou un trihaloborane tel que par exemple le trichlorure de bore ou le tribromure de bore, dans un solvant tel que le tétrahydrofurane.

Dans une variante du procédé, on prépare un organomagnésien à partir d'un composé de formule (2) dans laquelle Z représente un atome d'halogène, puis on procède comme décrit ci-dessus.

Les composés de départ sont disponibles dans le commerce ou décrits dans la littérature ou peuvent être préparés selon des méthodes qui y sont décrites ou qui sont connues de l'homme du métier.

Ainsi, le 2-(1,1-diméthyléthyl)-5-phényltétrazole est préparé selon la méthode décrite pour un dérivé analogue par J. W. TILLEY et coll. *J. Med. Chem. 1991*, **34**, 1125-1126.

Les dérivés de triarylborane ainsi obtenus, sont des solides stables que l'on peut coupler avec des halogénures aromatiques ou hétéroaromatiques comportant de nombreux substituants tels que par exemple, les groupes alkyle, hydroxyméthyle, aminométhyle, alcoxyméthyle, carboxamide, carbonyle, carboxyle, cyano, alcoxyle, nitro ou méthyle substitué par différents motifs hétérocycliques. Ces couplages peuvent être réalisés en milieu hydroorganique.

Le groupement 1,1-diméthyléthyle est un groupement protecteur particulièrement stable dans différentes conditions réactionnelles rencontrées en chimie organique. Son utilisation comme groupement protecteur de la fonction tétrazolyle, autorise l'emploi des composés de l'invention et des produits en résultant, dans différentes réactions de transformation chimique.

L'exemple qui suit illustre la préparation des composés selon l'invention.
Les microanalyses et les spectres IR et RMN confirment la structure des composés obtenus.

### Exemple

5,5',5''-[borylidynetris(1,2-phénylène)]tris[2-(1,1-diméthyléthyl)-2*H*-tétrazole]

Dans un ballon tricol de 100 ml maintenu sous atmosphère d'azote, on introduit 5 g (25 mmoles) de 2-(1,1-diméthyléthyl)-5-phényl-2*H*-tétrazole, 20 ml de tétrahydrofurane anhydre puis on ajoute goutte à goutte en 30 minutes, 13 ml d'une solution 2,5 M de *n*-butyllithium dans l'hexane. On porte le mélange à la température de reflux et on ajoute en 4 heures, 1,4 ml (12 mmoles) de triméthylborate. On évapore les solvants sous vide, on reprend le résidu dans 50 ml d'acétate d'éthyle et on le lave à l'eau. On évapore le solvant, on obtient un résidu huileux que l'on triture dans le méthanol et on récupère le précipité formé. On le filtre et on le sèche sous vide.
On obtient 3,5 g de produit.
Point de fusion = 230 °C

Les composés selon l'invention peuvent être utilisés pour la synthèse de composés répondant à la formule (I) 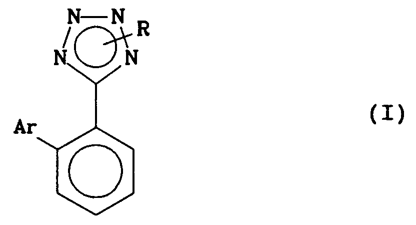
dans laquelle Ar représente soit un groupe aryle, soit un groupe hétéroaryle tel que par exemple les groupes pyridinyle ou pyrimidinyle, soit un groupe naphtyle, soit un groupe hétéroaryle condensé tel que par exemple les groupes quinoléine, isoquinoléine, phtalazine, cinnoline, quinazoline, benzofurane, benzothiophène, indole, benzothiazole, benzoxazole ou benzimidazole, éventuellement substitués par un groupe alkyle, aminométhyle, hydroxyméthyle, alcoxyméthyle, alcoxyle, carboxamide, carbonyle, carboxyle, cyano, nitro ou méthyle substitué par différents motifs hétérocycliques comme par exemple un groupe imidazolyle, pyridinyle, pyrimidinyle, imidazopyridinyle, triazolopyrimidinyle ou pyrazolopyrimidine, et R est tel que défini précédemment.

Les composés de formule (I) sont des intermédiaires dans la synthèse de composés antagonistes de l'angiotensine II tels que ceux décrits dans les demandes de brevets européens 0253310, 0324377, 0401030, 0407342, 0424317, 0500409, 0522038, 0540400 et dans le brevet européen 0521768.

La synthèse des composés de formule (I) à partir des composés de l'invention est réalisée selon l'une des méthodes décrites ci-après. On fait réagir un dérivé de formule (1) avec un dérivé de formule générale Ar-Z dans laquelle Z est un atome d'halogène ou un groupe -OSO₂CF₃ et Ar est tel que défini ci-dessus, en présence d'une base telle que par exemple, le carbonate de sodium, le carbonate de potassium, le dihydrogénophosphate de potassium ou une amine tertiaire comme la triéthylamine et d'un catalyseur à base de palladium complexé par une phosphine telle que la triphénylphosphine, la 1,2-bis-(diphénylphosphino)éthane ou la 1,2-bis-(diphénylphosphino)propane, dans un solvant tel que par exemple, le toluène, le xylène, le diméthylformamide, la *N*-méthylpyrrolidinone, le méthanol, l'éthanol, le butanol, l'isopropanol, le 1,1-diméthyléthanol ou l'alcool isoamylique purs ou en présence d'eau.

Les exemples ci-dessous illustrent la synthèse des composés de formule (I) à partir des composés de formule (1) de l'invention.

### A 2'[2-(1,1-diméthyléthyl)-2H-tétrazol-5-yl][1,1'-biphényl]-4-carboxaldéhyde

Dans un ballon tricol de 50 ml maintenu sous atmosphère d'azote, on introduit 1,1 g (1,79 mmoles) de 5,5',5''-[borylidynetris(1,2-phénylène)]tris[2-(1,1-diméthyléthyl)-2*H*-tétrazole] préparé selon la méthode décrite dans l'exemple précédent, 0,9 g (4,9 mmoles) de 4-bromobenzaldéhyde, 1,4 g de carbonate de potassium, 0,17 g (0,15 mmole) de palladiumtétrakis(triphénylphosphine), 20 ml de *N*,*N*-diméthylformamide et 2,5 ml d'eau. On chauffe le mélange à 85 °C pendant 1 heure puis on le verse sur 100 ml d'eau. Ensuite on extrait deux fois avec 50 ml d'acétate d'éthyle et on rassemble les phases organiques. On les lave à l'eau, on les sèche sur sulfate de sodium et on évapore le solvant à sec. On obtient un résidu huileux qui cristallise par trituration dans le cyclohexane. On récupère le solide ainsi obtenu, on le filtre et on le sèche sous vide.
On obtient 1,2 g de produit.
Point de fusion = 70 °C

### B 6-butyl-2-(2-phényléthyl)-5-[[2'[2-(1,1-diméthyléthyl)-2H-tétrazol-5-yl][1,1'-biphényl]-4-yl]méthyl]-pyrimidin-4(3H)one

### 1^{ère} méthode

Dans un ballon tricol de 50 ml maintenu sous atmosphère d'azote, on introduit 0,94 g (1,52 mmoles) de 5,5',5''-[borylidynetris(1,2-phénylène)]tris[2-(1,1-diméthyléthyl)-2*H*-tétrazole] préparé selon la méthode décrite précédemment, 1,7 g (4 mmoles) de 5-[(4-bromophényl)méthyl]-6-butyl-2-(2-phényléthyl)pyrimidin-4(3*H*)one, 0,9 g (8 mmoles) de 1,1-diméthyléthylate de potassium, 0,14 g (0,121 mmole) de palladiumtétrakis(triphénylphosphine) et 17 ml de 1,1-diméthyléthanol. On chauffe le mileu réactionnel pendant 10 heures à la température de reflux, on le dilue avec 25 ml d'eau et on extrait successivement par 40 ml puis par 20 ml d'acétate d'éthyle. On rassemble les phases organiques, on les lave avec de l'eau, on les sèche sur sulfate de sodium et on évapore le solvant à sec. On obtient un résidu huileux qui cristallise par trituration dans 5 ml de méthanol. On récupère le solide ainsi obtenu, on le filtre et on le sèche sous vide.
On obtient 1,43 g de produit.
Point de fusion = 144 °C

### 2^{ème} méthode

On chauffe à la température de reflux pendant 4 heures, 1 g (1,62 mmoles) de 5,5',5''-[borylidynetris(1,2-phénylène)]tris [2-(1,1-diméthyléthyl)-2*H*-tétrazole] préparé selon la méthode décrite précédemment, 2 g (5 mmoles) de 5-[(4-bromophényl) méthyl]-6-butyl-2-(2-phényléthyl)pyrimidin-4(3*H*)one, 0,163 g (0,140 mmole) de palladiumtétrakis(triphénylphosphine), 4,7 ml d'une solution 2 M de carbonate de sodium dans l'eau et 20 ml de toluène. On laisse le mélange refroidir, on élimine la phase aqueuse et on recueille la phase organique. On la lave avec de l'eau, on la sèche sur sulfate de sodium et on évapore le solvant à sec. On triture le résidu dans l'éthanol, on récupère le solide ainsi obtenu, on le filtre et on le sèche sous vide.
On obtient 1,1 g de produit sous forme d'un solide blanc.
Point de fusion = 143 °C

### 3^{ème} méthode

Dans un ballon tricol de 100 ml maintenu sous atmosphère d'azote, on introduit 1 g (1,62 mmoles) de 5,5',5''-[borylidynetris(1,2-phénylène)]tris[2-(1,1-diméthyléthyl)-2*H*-tétrazole] préparé selon la méthode décrite précédemment, 2 g (5 mmoles) de 5-[(4-bromophényl)méthyl]-6-butyl-2-(2-phényléthyl)pyrimidin-4(3*H*)one, 0,163 g (0,140 mmole) de palladiumtétrakis(triphénylphosphine), 1,3 g de carbonate de potassium, 20 ml de *N*,*N*-diméthylformamide et 5 ml d'eau. On chauffe le milieu réactionnel à 85 °C pendant 3 heures puis on le verse sur 100 ml d'eau. Ensuite on l'extrait par de l'acétate d'éthyle on lave la phase organique avec de l'eau, on la sèche sur sulfate de sodium et on évapore le solvant à sec. On obtient un résidu huileux qui cristallise par trituration dans le méthanol. On récupère le solide ainsi obtenu, on le filtre et on le sèche sous vide.
On obtient 1,7 g de produit sous forme d'un solide blanc.
Point de fusion = 145 °C

Le procédé selon l'invention permet d'obtenir des composés de formule (I) de haute pureté avec un bon rendement.
Il permet le couplage des halogénures aromatiques, hétéroaromatiques ou des analogues trifluorométhanesulfonyloxy comportant des fonctions organiques variées telles que par exemple les alcools, les éthers, les amines, les aldéhydes, les cétones, les acides, les esters, les nitriles, les amides, et les dérivés nitrés ou soufrés.
D'autre part ce procédé évite l'emploi d'azotures explosifs et contribue à la protection de l'environnement (recyclage du palladium).

## Revendications

1. Dérivés de triarylborane répondant à la formule (1) 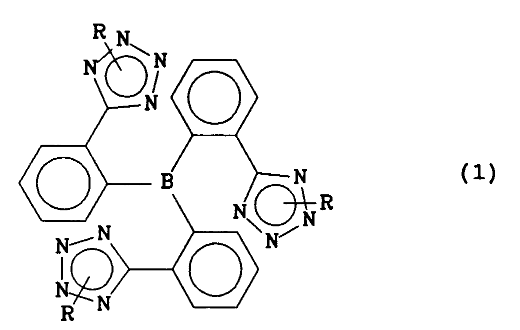 dans laquelle
R représente soit un groupe -CR₁R₂R₃ où R₁, R₂ et R₃ sont chacun indépendamment l'un de l'autre, un groupe (C₁-C₂)alkyle ou aryle, soit un groupe -CH₂OR₄ où R₄ est un groupe (C₁-C₂)alkyle ou benzyle, soit un groupe -Si(R₅)₃ où R₅ est un groupe (C₁-C₂) alkyle ou aryle, R étant en position 1 ou 2 du cycle tétrazole.

2. Dérivés de triarylborane selon la revendication 1 pour lesquels R représente un groupe -CR₁R₂R₃ où R₁, R₂ et R₃ sont chacun indépendamment l'un de l'autre un groupe (C₁-C₂)alkyle.

3. Le 5,5',5''-[borylidynetris(1,2-phénylène)]tris[2-(1,1-diméthyléthyl)-2*H*-tétrazole].

4. Procédé de préparation des dérivés selon la revendication 1, procédé caractérisé en ce que l'on fait réagir un dérivé de formule (2) 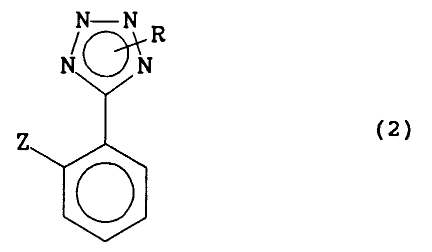 dans laquelle R est tel que défini dans la revendication 1 et Z représente soit un atome d'hydrogène, soit un atome d'halogène avec un dérivé lithié dans un solvant aprotique à une température comprise entre 20 °C et la température de reflux pour obtenir un organolithien que l'on fait réagir avec un tri(C₁-C₄)alkylborate ou un trihaloborane dans un solvant aprotique.

5. Procédé selon la revendication 4 caractérisé en ce que le dérivé lithié est un alkyllithium.

6. Procédé selon la revendication 4 caractérisé en ce que le dérivé lithié est l'hexaméthyldisilazane lithié.

7. Procédé de préparation des dérivés selon la revendication 1, procédé caractérisé en ce que l'on fait réagir un dérivé de formule (2) 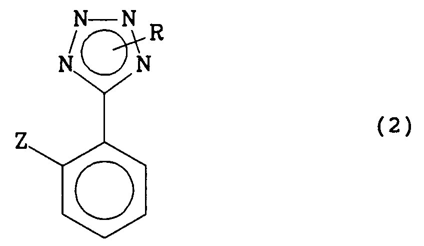 dans laquelle R est tel que défini dans la revendication 1 et Z représente un atome d'halogène avec du magnésium pour obtenir un organomagnésien que l'on fait réagir avec un tri(C₁-C₄)alkylborate ou un trihaloborane dans un solvant aprotique.

8. Utilisation des dérivés selon la revendication 1 pour la synthèse des composés répondant à la formule (I) 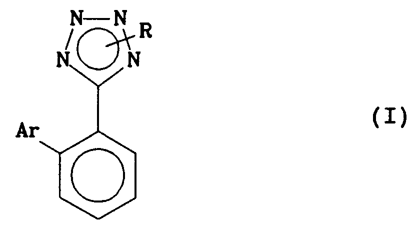 dans laquelle Ar représente soit un groupe aryle, soit un groupe hétéroaryle tel que par exemple les groupes pyridinyle ou pyrimidinyle, soit un groupe naphtyle, soit un groupe hétéroaryle condensé tel que par exemple les groupes quinoléine, isoquinoléine, phtalazine, cinnoline, quinazoline, benzofurane, benzothiophène, indole, benzothiazole, benzoxazole ou benzimidazole, éventuellement substitués par un groupe alkyle, aminométhyle,hydroxyméthyle, alcoxyméthyle, alcoxyle, carboxamide, carbonyle, carboxyle, cyano, nitro ou méthyle substitué par différents motifs hétérocycliques comme par exemple un groupe imidazolyle, pyridinyle, pyrimidinyle, imidazopyridinyle, triazolopyrimidinyle ou pyrazolopyrimidine, et R est tel que défini dans la revendication 1.
